# EUROPEAN PATENT SPECIFICATION

(11) **EP 3 076 957 B1**
(45) Date of publication and mention of the grant of the patent: **05.02.2020**
(21) Application number: 14819038.2
(22) Date of filing: 05.12.2014
(51) Int. Cl.: A61K 9/72, A61K 31/167, A61K 31/58, A61K 9/00, A61K 45/06, A61K 31/137, A61K 31/56, A61K 31/573

(54) **METHOD FOR PREPARING DRY POWDER INHALATION COMPOSITIONS**
VERFAHREN ZUR HERSTELLUNG VON TROCKENPULVERZUSAMMENSETZUNGEN ZUR INHALATION
PROCÉDÉ PERMETTANT DE PRÉPARER DES COMPOSITIONS D'INHALATION DE POUDRE SÈCHE

(30) Priority: 06.12.2013 US 201361913024 P
(43) Date of publication of application: 12.10.2016
(73) Proprietor: Orion Corporation, 02200 Espoo (FI)
(72) Inventor: MATTILA, Terhi, FI-70200 Kuopio (FI); HAPPONEN, Anita, FI-70340 Kuopio (FI); HAIKARAINEN, Jussi, FI-02200 Espoo (FI)
(74) Representative: J A Kemp LLP
(86) International application number: PCT/FI2014/000038
(87) International publication number: WO 2015/082756

(56) References cited:
- EP-A1- 2 221 048
- WO-A1-2011/145109
- WO-A1-2013/110632
- WO-A2-2004/017918

## Description

### Field of the Invention

The invention relates to a method for preparing dry powder inhalation compositions which comprise two or more active ingredients and inert particulate excipient, and to a method for adjusting the performance of such compositions.

### Background of the Invention

Inhaled medicaments for the treatment of respiratory diseases such as asthma and COPD are often formulated as dry powders and are delivered using a dry-powder inhaler (DPI). The medicaments are micronized such as to have a respirable aerodynamic diameter which is typically in the region of 0.5 to 10 µm. Such micronized particles tend to be cohesive and have poor flow properties. To increase flowability and dosing accuracy the fine drug particles of respirable size are typically mixed with coarser excipient particles to form an ordered mixture, wherein fine drug particles are attached to the coarser excipient particles. This technique necessitates the break-up of the drug/excipient agglomerates before they enter the patient's mouth and throat, where individual large particles and agglomerated large and small particles tend to deposit. Effective aerosolization and deagglomeration of the powder requires that forces exerted on particles (e. g. forces between particles and surfaces of the device, between drug particles and excipient particles or between drug particles themselves) must be overcome such that high fine particle dose (FPD) of medicament particles in the respirable size is obtained. Improvements in FPD and dose uniformity have been reported by suitably adjusting the size range of the excipient particles.

The interaction between the drug and the excipient is further complicated when the inhalation powder includes particles of two or more different active ingredients. In the development of such combination products it is often desirable to adjust the FPD level of each active ingredient independently. However, this often proves to be difficult as attempts to affect the FPD level of one active ingredient affect the FPD of the other active ingredient(s) of the combination as well.

Efforts to control the performance of dry powder inhalation products having two or more active ingredients have been recently reported. EP 2221048 discloses a method of first preblending each different active ingredient with different excipient (differing in their particle size) and, thereafter, blending the obtained preblends together in a main blending procedure to obtain the final composition. The method allegedly enables adhesion of each of the active ingredient to one excipient, but not to other. The FPD of one active substance could be then tuned independently from the other active substance by suitably adjusting the particle size of each excipient.

The method of EP 2221048 suffers from the drawback that various lactose grades with different particle size distribution are required for each tuning step. Commercial lactose grades may differ also in other characteristics than particle size and unexpected effects in the FPD may occur. Thus, there is a need for simplified methods for adjusting the FPD level of each active substance independently in inhaled dry powder combination products.

### Summary of the Invention

It has been found that FPD level of each active ingredient of the combination can be adjusted independently without the need to change the excipient grades used in the composition. This is achieved by providing two excipient grades which differ in their median particle size, mixing the first active ingredient and a portion of the second active ingredient with a first excipient to provide a first preblend, mixing the remaining portion of the second active ingredient with a second excipient to provide a second preblend, and finally mixing the first and the second preblends together. The FPD level of second active ingredient can be adjusted simply by changing the ratio how it is divided between the first and the second excipient. Preblending with coarser excipient is found to be associated with a lower FPD level. Thus, the FPD value of the second active ingredient can be adjusted to the desired level by changing the portion of second active ingredient which is preblended with coarser excipient. At the same time, the FPD level of the first active ingredient is only minimally affected.

### Brief Description of the Drawings

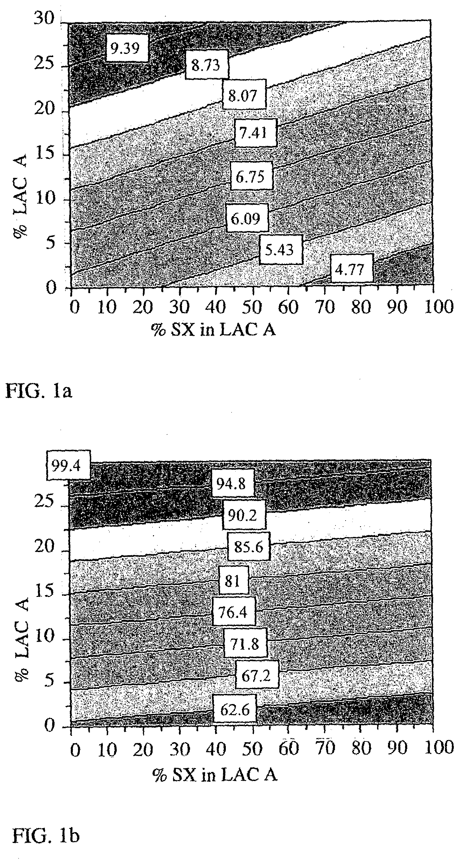

Figure 1a shows the observed changes in the fine particle dose (FPD) of salmeterol xinafoate by adjusting preblend parameters (percent of fine lactose and proportion of salmeterol xinafoate preblended with coarser excipient).
Figure 1b shows the observed changes in the FPD of fluticasone propionate in vitro using various preblend parameters (percent of fine lactose and proportion of salmeterol xinafoate preblended with coarser excipient).

### Detailed Description of the Invention

In one aspect, the present invention provides a method of preparing a dry powder inhalation composition comprising a first and a second active ingredient in micronized form comprising the steps of:
(a) mixing the first active ingredient and a portion of the second active ingredient with a first particulate excipient to provide a first preblend;
(b) mixing the remaining portion of the second active ingredient with a second particulate excipient to provide a second preblend; and
(c) mixing the first and the second preblends together;
wherein the first particulate excipient and the second particulate excipient differ in their median particle size, such that VMD (volume median diameter) of the finer particulate excipient is less than 90 % of the VMD of the coarser particulate excipient.

The term "about" refers to a variation of 5 % from the indicated numeric value.

The term "finer particulate excipient", as used herein, means the excipient selected from the first and the second particulate excipients which has the lowest VMD value.

The term "coarser particulate excipient", as used herein, means the excipient selected from the first and the second particulate excipients which has the highest VMD value.

The term "micronized form" means particle size lower than about 10 µm, for example in the range between 0.5 to 10 µm, particularly in the range between 1 and 6 µm.

The particle size, for example volume median diameter (VMD), of the particulate material can be determined by a laser diffractometer (e.g. Malvern Instruments Ltd, UK) using dry dispersion method and Fraunhofer approximation.

In another aspect, the present invention provides a method of preparing a dry powder inhalation composition comprising a first and a second active ingredient in micronized form comprising the steps of:
(a) mixing the first active ingredient and a portion of the second active ingredient with a first particulate excipient having VMD within the range of from 30 to 70 µm to provide a first preblend;
(b) mixing the remaining portion of the second active ingredient with a second particulate excipient having VMD within the range of from 80 to 150 µm to provide a second preblend; and
(c) mixing the first and the second preblends, optionally with additional first or second particulate excipient, together and
(d) optionally mixing the obtained blend with additional first or second particulate excipient.

The first and second active ingredients, which are understood herein to be different, can be generally any two active ingredients which are suitable for administration by inhalation in dry powders as a combination. In particular, the active ingredients may be selected from those that are useful in the treatment of respiratory diseases such as asthma and COPD. The present method can be used also in the preparation of dry powder inhalation compositions which incorporate more than two different active ingredients, for example in the preparation of a combination of three active ingredients.

According to one embodiment of the invention, the first and second active ingredients are selected from anti-inflammatory steroids and bronchodilators. Examples of anti-inflammatory steroids include, but are not limited to, budesonide, fluticasone, beclomethasone, ciclesonide, fluticasone, mometasone and pharmaceutically acceptable salts thereof. Examples of bronchodilators include, but are not limited to, formoterol, salmeterol, aclidinium, arformoterol, carmoterol, fenoterol, glycopyrronium, indacaterol, ipratropium, olodaterol, salbutamol, tiotropium, umeclidinium, vilanterol and pharmaceutically acceptable salts thereof.

According to one particular embodiment of the invention, the first active ingredient is an anti-inflammatory steroid and the second active ingredient is a bronchodilator. According to another particular embodiment of the invention, the first active ingredient is a bronchodilator and the second active ingredient is an anti-inflammatory steroid. According to another particular embodiment of the invention, the first active ingredient is budesonide or a pharmaceutically acceptable salt thereof and the second active ingredient is formoterol or a pharmaceutically acceptable salt thereof. According to still another particular embodiment of the invention, the first active ingredient is fluticasone or a pharmaceutically acceptable salt thereof and the second active ingredient is salmeterol or a pharmaceutically acceptable salt thereof.

The active ingredients should be in micronized form, i.e. having particle size lower than about 10 µm, for example in the range from about 0.5 to about 10 µm, particularly in the range from about 1 to about 6 µm, such as to be able to deposit target areas in the lungs. Conventional methods, such as milling, can be used to provide the active ingredients in micronized form.

The amount of the active ingredient in the dry powder inhalation composition can vary depending e.g. on the active ingredient and the type of dry powder inhaler used. Generally, the amount of the active ingredient in the dry powder inhalation composition is within the range of 0.02 to 30 %, typically from 0.05 to 10 %, more typically from 0.1 to 5 %, per weight of the composition.

According to one embodiment of the invention, the excipient used in the dry powder inhalation composition is a mono- or disaccharide, particularly lactose or mannitol, for example alpha lactose monohydrate. In general, the particle size of the excipient is preferably such that it can be entrained in the air stream but not enter deeply into the lung. However, a small proportion of particles with respirable size (< 10 µm) can be present in the excipient as such fine particles of the excipient may help in attaining higher FPD values. The VMD of the excipient, such as lactose, to be used in the composition is suitably in the range of, for example, from about 30 to about 150 µm. The excipient of desired VMD can be obtained from commercial sources or can be prepared using methods known in the art such as by blending together excipient powders of known particle size or by sieving.

In the present method two particulate excipients are used which differ in their median particle size such that the VMD of the finer particulate excipient is less than 90 % of the VMD of the coarser particulate excipient. According to one embodiment of the invention, the VMD of the finer particulate excipient is less than 85 % of the VMD of the coarser particulate excipients. According to another embodiment of the invention, the VMD of the finer particulate excipient is within the range of from 30 to 70 µm, for example from about 35 to about 65 µm, and the VMD of the coarser particulate excipient is within the range of from about 80 to about 150 µm, for example from about 90 to about 120 µm.

The weight ratio of the finer particulate excipient to coarser particulate excipient in the dry powder inhalation composition may vary within a wide range, but is typically within the range of 0.2 to 5, more typically from 0.25 to 3, for example from 0.5 to 1.5.

Preferably, the first and the second particulate excipient is lactose.

In the first step of the mixing process, the entirety of the first active ingredient and a portion of the second active ingredient are mixed with a first particulate excipient (e.g. the finer particulate excipient) to provide a first preblend. In this step the entirety or only a portion of the total first excipient may be used. If only a proportion of the first excipient is used in this step, the proportion is typically 5 - 80 %, for example 10-75 %, of the total amount used in the composition. The rest of the first particulate excipient may then be used in subsequent blending steps. The components of the first preblend are mixed in a suitable blending device, for example a low shear powder mixer or high shear powder mixer. The mixing speed and mixing time may vary within a broad range depending on the blending device used but are in general selected such as to produce homogenous powder composition. The mixing speed may be for example in the range of 10 - 50 rpm, for example 20 - 40 rpm. The mixing time may be for example in the range of 1 to 60 min, for example 3 to 15 min.

The second preblend is prepared by mixing the remaining portion of the second active ingredient with a second particulate excipient (e.g. the coarser particulate excipient) to provide a second preblend. In this step the entirety or only a portion of the total second excipient may be used. If only a proportion of the second excipient is used in this step, the proportion is typically 1-50 %, for example 1-30 %, of the total amount used in the composition. The rest of the second particulate excipient may then be used in the final blending step. The same blending conditions as above can be utilized.

Next the first and the second preblends are suitably sieved and then mixed together. If only a portion of the first excipient was used in the preparation of the first preblend, the rest of the first excipient (e.g. the rest of the finer particulate excipient) is mixed in this step with the first and the second preblends to obtain a blend containing the first and the second active ingredient. The blend is then preferably sieved. The same blending conditions as above can be utilized.

If only a portion of the second excipient was used in the preparation of the second preblend, the rest of the second excipient (e.g. the rest of the coarser particulate excipient) can now be mixed with the previously obtained blend containing the first and the second active ingredient such as to obtain the final dry powder inhalation composition. The same blending conditions as above can be utilized. The final composition can be filled in a suitable dry powder inhaler.

If desired, further excipients or active ingredients can be added to the composition during above mentioned mixing steps or during further mixing steps.

In the development of dry powder inhalation compositions incorporating a combination of active ingredients there often occurs a desire to adjust the fine particle dose (FPD) of one active ingredient independently of the other active ingredient. According to the present method, the FPD of the second active ingredient of the composition can be independently adjusted by simply changing the proportion of the second active ingredient which is mixed with a first particulate excipient. Preblending the active ingredient with coarser excipient is found to be associated with lower FPD level. Thus, in case the first particulate excipient is the finer particulate excipient, the FPD of the second active ingredient can be increased by increasing the proportion of the second active ingredient which is mixed with a first particulate excipient. Inversely, the FPD of the second active ingredient can be decreased by decreasing the proportion of the second active ingredient which is mixed with the first particulate excipient.

The invention is illustrated further with the following Examples.

### Example 1.

A dry powder formulation for inhalation according to Table 1 was prepared.

**Table 1. Dry powder formulation for inhalation**

| | |
|---|---|
| Formoterol fumarate dihydrate | 1.71 g |
| Budesonide | 60.9 g |
| Lactose A (VMD = 55 µm) | 862 g |
| Lactose B (VMD = 105 µm) | 575 g |

A first preblend was prepared by mixing 0.855 g of micronized formoterol fumarate dihydrate, 60.9 g of micronized budesonide and 287 g of lactose A in a swing-mix type powder mixer followed by sieving the mixture and blending it with the rest (575 g) of lactose A in a swing- mix type powder mixer. A second preblend was prepared by mixing 0.855 g of micronized formoterol fumarate dihydrate and 192 g of lactose B in a swing-mix type powder mixer followed by sieving the mixture and blending it with 192 g of lactose B in a swing-mix type powder mixer. The two preblends were combined by sieving and then mixed with the rest (191 g) of lactose B in a swing-mix type powder mixer to obtain the final formulation. The mixing time of each step was 5 min at 35 rpm.

### Example 2. (Reference example)

A dry powder formulation for inhalation according to Example 1 was prepared with the exception that all formoterol fumarate dihydrate was incorporated in the second preblend (with lactose B).

### Example 3. (Reference example)

A dry powder formulation for inhalation according to Example 1 was prepared with the exception that all formoterol fumarate dihydrate was incorporated in the first preblend (with lactose A).

### Example 4.

The formulations of Examples 1, 2 and 3 were filled in Easyhaler® powder inhaler device and the FPD values for the active ingredients (formoterol fumarate dihydrate and budesonide) were determined in vitro using methods well known in the art. The results are shown in Table 2.

**Table 2. Fine particle dose (FPD) values of formulations of Examples 1-3.**

| Example No. | FPD of formoterol (µg/dose) | FPD of budesonide (µg/dose) |
|---|---|---|
| 2 | 2.8 | 137 |
| 1 | 3.1 | 139 |
| 3 | 3.6 | 133 |

It can be seen that FPD of formoterol fumarate dihydrate can be adjusted by blending part of formoterol fumarate dihydrate with lactose B (coarser lactose) while the FPD of budesonide is only minimally affected. The change in FPD is dependent on the proportion of formoterol which is blended with lactose B (coarser lactose).

### Example 5.

A dry powder formulation for inhalation according to Table 3 was prepared.

**Table 3. Dry powder formulation for inhalation**

| | |
|---|---|
| Salmeterol xinafoate | 1.9 g |
| Fluticasone propionate | 13.0 g |
| Lactose A (VMD = 55 µm) | 145.5 g |
| Lactose B (VMD = 105 µm) | 339.6 g |

A first preblend was prepared by mixing 0.95 g of micronized salmeterol xinafoate, 13.0 g of micronized fluticasone propionate and 107.3 g of lactose A in a swing- mix type powder mixer. A second preblend was prepared by mixing 0.95 g of micronized salmeterol xinafoate and 7.3 g of lactose B in a swing- mix type powder mixer. The two preblends were combined by sieving and then mixed with 38.2 g of lactose A in a swing- mix type powder mixer. The resulting mixture was sieved and finally mixed with 332.3 g of lactose B in a swing- mix type powder mixer to obtain the final formulation. The mixing time of each step was 5 min at 35 rpm.

### Example 6. Effect of salmeterol/lactose ratio of the second preblend on fine particle dose (FPD)

It was studied how changes in the ratio of salmeterol/lactose B in the second preblend affects the fine particle dose (FPD) of the final salmeterol/fluticasone formulation. A series of salmeterol/fluticasone formulations were prepared as in the previous example with the exception that the proportion of salmeterol used in the second preblend was varied as well as the proportion of lactose A (of the total lactose) used in the formulation. The formulations were filled in Easyhaler® powder inhaler device and the FPD values for the active ingredients were determined in vitro using methods well known in the art. The results were fitted in a mathematical model which demonstrates the effect of the variables on the FPD of the active ingredients. The observed changes in the FPD of salmeterol xinafoate and fluticasone propionate are presented in Figure 1a and 1b, respectively. In the Figures the proportion (% of total) of salmeterol xinafoate (SX) mixed with lactose A (finer lactose) in the first preblend is shown in x-axis. The proportion of lactose A (% of total lactose) used in the composition is shown in y-axis. The resulting FPD value of salmeterol xinafoate is shown in Figure 1a as zones ranging from about 4 µg/dose to about 10 µg/dose. The resulting FPD value of fluticasone propionate is shown similarly in Figure 1b.

The Figures demonstrate that the FPD value (shown in a box) for each active ingredient of the composition can be increased by increasing the proportion of lactose A (finer lactose) in the composition. For example, the FPD of salmeterol can be increased from about 4 µg/dose to about 10 µg/dose by increasing the proportion of lactose A (finer lactose) from 0 % to 30 % (Figure 1a). At the same time, the FPD of fluticasone increases from about 60 µg/dose to about 100 µg/dose (Figure 1b).

On the other hand, when part of salmeterol of the composition is blended with lactose B (coarser lactose), the FPD value of salmeterol is decreased. The decrease is dependent on the proportion of salmeterol which is blended with lactose B (coarser lactose). For example, when the ratio of lactose A to lactose B is fixed to 10:90 (i.e. proportion of lactose A is 10 %) in the composition, the FPD of salmeterol can be linearly decreased from about 7.2 µg/dose down to about 5.5 µg/dose by increasing the part of salmeterol which is blended with lactose B (coarser lactose) from 0 % to 100 %. This corresponds to a decrease of 24 % in the FPD of salmeterol. Importantly, at the same time the FPD value of fluticasone drops only 5 % (from about 74 µg/dose to about 70 µg/dose). Thus, blending part of the second active ingredient of the combination with a second lactose grade differing in the particle size from the first lactose grade provides a method of adjusting the FPD of the second active ingredient while only minimally affecting the FPD of the first active ingredient of the composition.

## Claims

1. A method of preparing a dry powder inhalation composition comprising a first and a second active ingredient in micronized form comprising the steps of:
(a) mixing the first active ingredient and a portion of the second active ingredient with a first particulate excipient to provide a first preblend;
(b) mixing the remaining portion of the second active ingredient with a second particulate excipient to provide a second preblend; and
(c) mixing the first and the second preblends together;
wherein the first particulate excipient and the second particulate excipient differ in their median particle size, such that VMD (volume median diameter) of the finer particulate excipient is less than 90 % of the VMD of the coarser particulate excipient.

2. A method according to claim 1, wherein the VMD of the finer particulate excipient is less than 85 % of the VMD of the coarser particulate excipient.

3. A method according to claim 1 or 2, wherein the VMD of the finer particulate excipient is within the range of from 30 to 70 µm and the VMD of the coarser particulate excipient is within the range of from 80 to 150 µm.

4. A method of preparing a dry powder inhalation composition comprising a first and a second active ingredient in micronized form comprising the steps of:
(a) mixing the first active ingredient and a portion of the second active ingredient with a first particulate excipient having VMD within the range of from 30 to 70 µm to provide a first preblend;
(b) mixing the remaining portion of the second active ingredient with a second particulate excipient having VMD within the range of from 80 to 150 µm to provide a second preblend; and
(c) mixing the first and the second preblends, optionally with additional first or second particulate excipient, together and
(d) optionally mixing the obtained blend with additional first or second particulate excipient.

5. A method according to any of claims 1 to 4, wherein the first and second particulate excipient is lactose.

6. A method according to any of claims 1 to 5, wherein the first and second active ingredients are selected from anti-inflammatory steroids and bronchodilators.

7. A method according to any of claims 1 to 6, wherein the first active ingredient is an anti-inflammatory steroid and the second active ingredient is a bronchodilator.

8. A method according to any of claims 1 to 6, wherein the first active ingredient is a bronchodilator and the second active ingredient is an anti-inflammatory steroid.

9. A method according to any of claims 1 to 8, wherein the anti-inflammatory steroid is budesonide, fluticasone, beclomethasone or a pharmaceutically acceptable salt thereof.

10. A method according to any of claims 1 to 9, wherein the bronchodilator is formoterol, salmeterol or a pharmaceutically acceptable salt thereof.

11. A method according to claim 7, wherein the first active ingredient is budesonide or a pharmaceutically acceptable salt thereof and the second active ingredient is formoterol or a pharmaceutically acceptable salt thereof.

12. A method according to claim 7, wherein the first active ingredient is fluticasone or a pharmaceutically acceptable salt thereof and the second active ingredient is salmeterol or a pharmaceutically acceptable salt thereof.

13. A method according to claim 7, wherein the first active ingredient is beclomethasone or a pharmaceutically acceptable salt thereof and the second active ingredient is formoterol or a pharmaceutically acceptable salt thereof.

## Patentansprüche

1. Verfahren zur Herstellung einer Trockenpulver-Inhalationszusammensetzung, umfassend einen ersten und einen zweiten Wirkstoff in mikronisierter Form, umfassend die Schritte:
(a) Mischen des ersten Wirkstoffs und eines Teils des zweiten Wirkstoffs mit einem ersten partikelförmigen Hilfsstoff, um eine erste Vormischung bereitzustellen;
(b) Mischen des verbleibenden Teils des zweiten Wirkstoffs mit einem zweiten partikelförmigen Hilfsstoff, um eine zweite Vormischung bereitzustellen; und
(c) Mischen der ersten und der zweiten Vormischung miteinander;
wobei der erste partikelförmige Hilfsstoff und der zweite partikelförmige Hilfsstoff sich in ihrer mittleren Partikelgröße unterscheiden, so dass der VMD (mittlerer Volumendurchmesser) des feineren partikelförmigen Hilfsstoffs weniger als 90 % der VMD des gröberen partikelförmigen Hilfsstoffs beträgt.

2. Verfahren nach Anspruch 1, wobei der VMD des feineren partikelförmigen Hilfsstoffs weniger als 85 % der VMD des gröberen partikelförmigen Hilfsstoffs beträgt.

3. Verfahren nach Anspruch 1 oder 2, wobei der VMD des feineren partikelförmigen Hilfsstoffs im Bereich von 30 bis 70 µm liegt und der VMD des gröberen partikelförmigen Hilfsstoffs im Bereich von 80 bis 150 µm liegt.

4. Verfahren zur Herstellung einer Trockenpulver-Inhalationszusammensetzung, umfassend einen ersten und einen zweiten Wirkstoff in mikronisierter Form, umfassend die Schritte:
(a) Mischen des ersten Wirkstoffs und eines Teils des zweiten Wirkstoffs mit einem ersten partikelförmigen Hilfsstoff mit einem VMD im Bereich von 30 bis 70 µm, um eine erste Vormischung bereitzustellen;
(b) Mischen des verbleibenden Teils des zweiten Wirkstoffs mit einem zweiten partikelförmigen Hilfsstoff mit einem VMD im Bereich von 80 bis 150 µm, um eine zweite Vormischung bereitzustellen; und
(c) Mischen der ersten und der zweiten Vormischungen, optional mit zusätzlichem ersten oder zweiten partikelförmigen Hilfsstoff, und
(d) optional Mischen der erhaltenen Mischung mit zusätzlichem ersten oder zweiten partikelförmigen Hilfsstoff.

5. Verfahren nach einem der Ansprüche 1 bis 4, wobei der erste und der zweite partikelförmige Hilfsstoff Lactose ist.

6. Verfahren nach einem der Ansprüche 1 bis 5, wobei der erste und der zweite Wirkstoff aus entzündungshemmenden Steroiden und Bronchodilatatoren ausgewählt sind.

7. Verfahren nach einem der Ansprüche 1 bis 6, wobei der erste Wirkstoff ein entzündungshemmendes Steroid ist und der zweite Wirkstoff ein Bronchodilatator ist.

8. Verfahren nach einem der Ansprüche 1 bis 6, wobei der erste Wirkstoff ein Bronchodilatator ist und der zweite Wirkstoff ein entzündungshemmendes Steroid ist.

9. Verfahren nach einem der Ansprüche 1 bis 8, wobei das entzündungshemmende Steroid Budesonid, Fluticason, Beclomethason oder ein pharmazeutisch annehmbares Salz davon ist.

10. Verfahren nach einem der Ansprüche 1 bis 9, wobei der Bronchodilatator Formoterol, Salmeterol oder ein pharmazeutisch annehmbares Salz davon ist.

11. Verfahren nach Anspruch 7, wobei der erste Wirkstoff Budesonid oder ein pharmazeutisch annehmbares Salz davon ist und der zweite Wirkstoff Formoterol oder ein pharmazeutisch annehmbares Salz davon ist.

12. Verfahren nach Anspruch 7, wobei der erste Wirkstoff Fluticason oder ein pharmazeutisch annehmbares Salz davon ist und der zweite Wirkstoff Salmeterol oder ein pharmazeutisch annehmbares Salz davon ist.

13. Verfahren nach Anspruch 7, wobei der erste Wirkstoff Beclomethason oder ein pharmazeutisch annehmbares Salz davon ist und der zweite Wirkstoff Formoterol oder ein pharmazeutisch annehmbares Salz davon ist.

## Revendications

1. Procédé de préparation d'une composition pour inhalation à base de poudre sèche comprenant un premier et un second ingrédient actif sous forme micronisée, comprenant les étapes consistant à :
(a) mélanger le premier ingrédient actif et une partie du second ingrédient actif avec un premier excipient particulaire pour fournir un premier pré-mélange ;
(b) mélanger la partie restante du second ingrédient actif avec un second excipient particulaire pour fournir un second pré-mélange ; et
(c) mélanger les premier et second pré-mélanges ensemble ;
dans lequel le premier excipient particulaire et le second excipient particulaire diffèrent par leur taille de particule médiane, de telle sorte que le VMD (diamètre médian en volume) de l'excipient particulaire plus fin est inférieur à 90% du VMD de l'excipient particulaire plus grossier.

2. Procédé selon la revendication 1, dans lequel le VMD de l'excipient particulaire plus fin est inférieur à 85% du VMD de l'excipient particulaire plus grossier.

3. Procédé selon la revendication 1 ou 2, dans lequel le VMD de l'excipient particulaire plus fin se situe dans la plage de 30 à 70 µm et le VMD de l'excipient particulaire plus grossier se situe dans la plage de 80 à 150 µm.

4. Procédé de préparation d'une composition pour inhalation à base de poudre sèche comprenant un premier et un second ingrédient actif sous forme micronisée comprenant les étapes consistant à :
(a) mélanger le premier ingrédient actif et une partie du second ingrédient actif avec un premier excipient particulaire ayant un VMD dans la plage de 30 à 70 µm pour fournir un premier pré-mélange ;
(b) mélanger la partie restante du second ingrédient actif avec un second excipient particulaire ayant un VMD dans la plage de 80 à 150 µm pour former un second pré-mélange ; et
(c) mélanger les premier et second pré-mélanges, éventuellement avec un premier ou un second excipient particulaire supplémentaire, ensemble et
(d) mélanger éventuellement le mélange obtenu avec un premier ou un second excipient particulaire supplémentaire.

5. Procédé selon l'une quelconque des revendications 1 à 4, dans lequel les premier et second excipients particulaires sont du lactose.

6. Procédé selon l'une quelconque des revendications 1 à 5, dans lequel les premier et second ingrédients actifs sont choisis parmi les stéroïdes anti-inflammatoires et les bronchodilatateurs.

7. Procédé selon l'une quelconque des revendications 1 à 6, dans lequel le premier ingrédient actif est un stéroïde anti-inflammatoire et le second ingrédient actif est un bronchodilatateur.

8. Procédé selon l'une quelconque des revendications 1 à 6, dans lequel le premier ingrédient actif est un bronchodilatateur et le second ingrédient actif est un stéroïde anti-inflammatoire.

9. Procédé selon l'une quelconque des revendications 1 à 8, dans lequel le stéroïde anti-inflammatoire est le budésonide, la fluticasone, la béclométhasone ou un de ses sels pharmaceutiquement acceptables.

10. Procédé selon l'une quelconque des revendications 1 à 9, dans lequel le bronchodilatateur est le formotérol, le salmétérol ou un de ses sels pharmaceutiquement acceptables.

11. Procédé selon la revendication 7, dans lequel le premier ingrédient actif est le budésonide ou un de ses sels pharmaceutiquement acceptables et le second ingrédient actif est du formotérol ou un de ses sels pharmaceutiquement acceptables.

12. Procédé selon la revendication 7, dans lequel le premier ingrédient actif est la fluticasone ou un de ses sels pharmaceutiquement acceptables et le second ingrédient actif est du salmétérol ou un de ses sels pharmaceutiquement acceptables.

13. Procédé selon la revendication 7, dans lequel le premier ingrédient actif est le béclométhasone ou un de ses sels pharmaceutiquement acceptables et le second ingrédient actif est le formotérol ou un de ses sels pharmaceutiquement acceptables.
